# EUROPEAN PATENT SPECIFICATION

(11) **EP 3 287 554 B1**
(45) Date of publication and mention of the grant of the patent: **02.10.2019**
(21) Application number: 16817663.4
(22) Date of filing: 07.06.2016
(51) Int. Cl.: D04H 1/70, A41B 13/04, A61F 13/511, A61F 13/513

(54) **NONWOVEN FABRIC AND ABSORBENT ARTICLE USING SAME**
VLIESSTOFF UND SAUGFÄHIGER ARTIKEL MIT VERWENDUNG DAVON
TEXTILE NON-TISSÉ ET ARTICLE ABSORBANT L'UTILISANT

(30) Priority: 30.06.2015 JP 2015132160
(43) Date of publication of application: 28.02.2018
(73) Proprietor: Unicharm Corporation, Ehime 799-0111 (JP)
(72) Inventor: KIMURA, Akihiro, Kanonji-shi Kagawa 769-1602 (JP); DETANI, Ko, Kanonji-shi Kagawa 769-1602 (JP); KURITA, Noritomo, Kanonji-shi Kagawa 769-1602 (JP)
(74) Representative: Krauns, Christian
(86) International application number: PCT/JP2016/066957
(87) International publication number: WO 2017/002554

(56) References cited:
- CN-U- 203 841 923
- JP-A- H08 100 360
- JP-A- 2004 113 489
- JP-A- 2007 154 359
- JP-A- 2009 030 218
- JP-A- 2009 072 398
- JP-A- 2009 153 731
- JP-A- 2012 125 945
- JP-A- 2012 149 370
- JP-A- 2014 012 913
- JP-A- 2014 083 228
- US-A1- 2014 121 626

## Description

### Technical Field

The present invention relates to a nonwoven fabric, and particularly a nonwoven fabric comprising a plurality of protrusions on the surface, and an absorbent article using it.

### Background Art

Absorbent articles such as disposable diapers and sanitary napkins are usually used in direct contact with the skin of the user, and some are even worn for long periods. Absorbent articles are therefore desired to have excellent cushioning properties and flexibility, and a satisfactory feel on the skin.

As for such an absorbent article, PTL 1 discloses a nonwoven fabric to be used as the top sheet of an absorbent article, the nonwoven fabric having first protrusions protruding on a first surface side and second protrusions protruding on a second surface side, which alternately spread out in two directions, a first direction and a second direction, within the plane, and connected through wall sections, wherein the wall sections form a ring structure, and the fiber density of the first protrusions is lower than the fiber density of the second protrusions. With this nonwoven fabric, the fluid drawing property is satisfactory, it has a soft cushioning property, good restoration after pressing and particularly excellent excreta-collecting properties.

The CN 203841923 U discloses a three-dimensional nonwoven fabric with a pore structure including raised regions, recessed regions, and the pore structure. The fiber densities from the raised regions to the recessed regions exhibit a gradient. The fiber density of the edges transiting from the raised regions to the recessed regions is higher than that of the tops of the raised regions. The pressure resistance performance of the high-density fiber regions keeps the three-dimensional nonwoven fabric significantly uneven, and quickens liquid permeation and reduces residues, and the three-dimensional nonwoven fabric has a high permeating speed.

### Citation List

### Patent Literature

[PTL 1] Japanese Unexamined Patent Publication No. 2012-136791

### Summary of Invention

### Technical Problem

However, because the nonwoven fabric disclosed in PTL 1 has relatively low fiber density at the wall sections, when the nonwoven fabric is used as a top sheet in an absorbent article, the protrusions have high collapsability under pressure such as body pressure of the wearer, and in some cases it has not been possible to adequately exhibit the satisfactory cushioning properties of the protrusions for long periods. In addition, since the nonwoven fabric disclosed in PTL 1 has relatively high fiber density at the top sections of the protrusions, and the top sections of the protrusions are rounded and in point-contact with the skin surface, the hardness of the protrusions tends to be felt and a satisfactory feel on the skin cannot be obtained in some cases.

It is therefore an object of the present invention to provide a nonwoven fabric that can adequately exhibit satisfactory cushioning properties for long periods, and that has a satisfactory feel on the skin.

### Solution to Problem

The nonwoven fabric according to one aspect (aspect 1) of the present invention is a nonwoven fabric comprising a base having a mutually opposite first surface and second surface and extending out flat, and a plurality of protrusions protruding from the first surface of the base in the thickness direction, wherein each of the protrusions comprises a peripheral surface section standing up from the first surface of the base in a thickness direction and a top surface section having a top surface forming a crown of the each of the protrusions, the fiber density of the peripheral surface section is higher than the fiber density of the top surface section, and at least a part of a top surface perimeter portion of the top surface section is constructed as a thickest section which has the greatest thickness in the nonwoven fabric. The top surface section is constructed so that a top surface perimeter portion located on one side in a machine direction or an orthogonal direction with respect to the machine direction in the planar view is constructed as a section with greater thickness, while the top surface perimeter portion located on the other side in the machine direction or an orthogonal direction is constructed as a section with smaller thickness.

According to the nonwoven fabric of aspect 1, the construction is such that the fiber density of the peripheral surface section that stand up in the thickness direction of the protrusion is higher than the fiber density of the top surface section, and therefore even when pressure by body pressure or the like is applied in the thickness direction of the nonwoven fabric, since the peripheral surface section functions as columns supporting the top surface section, the protrusion is less likely to collapse, and even if the top surface section of the protrusion collapses, since at least a part of the top surface perimeter portion of the top surface section is constructed as the thickest section having the greatest thickness in the nonwoven fabric, it is possible to restore collapse (deformation) of the top surface section by the resiliency of the thickest section. This allows the nonwoven fabric of aspect 1 to adequately exhibit the satisfactory cushioning properties of the protrusions for long periods.

In addition, since the thickest section has satisfactory resiliency and flexibility, even if the protrusion has the peripheral surface section with high fiber density, the hardness of the protrusion is less likely to be felt and a satisfactory feel on the skin can be provided.

Furthermore, according to another aspect (aspect 2) of the present invention, in the nonwoven fabric of aspect 1, the construction is such that the fiber density of the peripheral surface section is higher than the fiber density of the base.

According to the nonwoven fabric of aspect 2, the construction is such that the fiber density of the peripheral surface section is higher than the fiber density of the base, and therefore pressure in the thickness direction that is applied from the first surface side of the protrusion by body pressure or the like can be buffered by bending of the base on the second surface side. Thus, the protrusions will be even less likely to collapse, and excellent flexibility can be imparted to the nonwoven fabric.

Furthermore, according to another aspect (aspect 3) of the present invention, in the nonwoven fabric of aspect 1 or 2, the top surface of the top surface section is depressed.

According to the nonwoven fabric of aspect 3, the top surface of the top surface section is depressed, and therefore even when pressure has been applied in the thickness direction from the first surface side of the protrusion by body pressure or the like, since the pressure is applied in a manner centered on the section of the top surface supported by the peripheral surface section (i.e., the top surface perimeter portion), it is less likely for the top surface to deform first and thus cause the protrusion as a whole to collapse.

Furthermore, according to another aspect (aspect 4) of the present invention, in the nonwoven fabric of any of aspects 1 to 3, a planar shape of the top surface of the top surface section is circular.

According to the nonwoven fabric of aspect 4, the planar shape of the top surface of the top surface section is circular, and the top surface is uniformly supported along the top surface perimeter portion by the peripheral surface section, and therefore high collapsable section is unlikely to form locally in the protrusion, and the protrusion is even less likely to collapse.

Furthermore, according to another aspect (aspect 5) of the present invention, in the nonwoven fabric according to any of aspects 1 to 4, the thickest section has the lowest fiber density in the nonwoven fabric.

According to the nonwoven fabric of aspect 5, the thickest section of the top surface section of the protrusion has the lowest fiber density in the nonwoven fabric, and therefore the resiliency and flexibility of the top surface perimeter portion is relatively high, and even when the top surface section of the protrusion collapses due to pressure such as body pressure, it is possible to more reliably restore collapse (deformation) of the top surface section. In addition, since the top surface perimeter portion has relatively high resiliency and flexibility, the hardness of the protrusions is even less likely to be felt, and satisfactory feel on the skin can be obtained.

Yet another aspect (aspect 6) of the present invention is an absorbent article including the nonwoven fabric according to any of aspects 1 to 5.

The absorbent article of aspect 6 includes a nonwoven fabric according to any of aspects 1 to 5, which can adequately exhibit satisfactory cushioning properties for long periods and has satisfactory feel on the skin, and therefore the wearer of the absorbent article can be provided with an excellent feel during wear for long periods.

### Advantageous Effects of Invention

According to the present invention it is possible to provide a nonwoven fabric that can adequately exhibit satisfactory cushioning properties for long periods, and that has a satisfactory feel on the skin.

### Brief Description of Drawings

Fig. 1 is a perspective view of a disposable diaper using a nonwoven fabric according to an embodiment of the present invention as the top sheet.
Fig. 2 is a plan view of the disposable diaper of Fig. 1 in the deployed state.
Fig. 3 is a partial plan view of a nonwoven fabric according to an embodiment of the present invention.
Fig. 4 is a partial end view of an end face along line IV-IV of Fig. 3.
Fig. 5 is a schematic view for illustration of thickness distribution in the top surface section of a protrusion of a nonwoven fabric according to an embodiment of the present invention.
Fig. 6 is a schematic view for illustration of thickness distribution in the top surface section of a protrusion of a nonwoven fabric according to another embodiment of the present invention.
Fig. 7 is an end view of an end face along line VII-VII of Fig. 6.
Fig. 8 is a schematic diagram of a production apparatus for production of a nonwoven fabric according to an embodiment of the present invention.
Fig. 9 is a magnified schematic view of section X in Fig. 8.
Fig. 10(a) is a photomicrograph of a cross-section along the center axis line in the MD direction at a section of a protrusion of the nonwoven fabric of Example 1 at the downstream end in the MD direction (a line corresponding to the center axis line VW in Fig. 5), and Fig. 10(b) is a photomicrograph of a cross-section along the center axis line in the MD direction at a section of a protrusion of the nonwoven fabric of Example 1 at the upstream end in the MD direction.
Fig. 11 is a photomicrograph of a cross-section along the center axis line in the MD direction of a protrusion of the nonwoven fabric of Comparative Example 1.

### Description of Embodiments

Preferred embodiments of the nonwoven fabric of the present invention will now be explained in greater detail with reference to the accompanying drawings. Throughout the present description, unless otherwise specified, the concept of "an object placed on a horizontal surface in the deployed state being viewed in the thickness direction of the object from the top side in the vertical direction" will be referred to simply by the term "planar view", and "the shape in the planar view" will be referred to simply as "the planar shape".

Furthermore, throughout the present description, the widthwise direction D_{W} is the widthwise direction (short direction) in the planar view, for a disposable diaper 1 (absorbent article) in the deployed state, the lengthwise direction D_{L} is the lengthwise direction (front-back direction of the wearer) in the planar view for the disposable diaper 1 (absorbent article) in the deployed state, and the thickness direction D_{T} is the thickness direction for the disposable diaper 1 (absorbent article) in the deployed state, the widthwise direction D_{W}, lengthwise direction D_{L} and thickness direction D_{T} being in a mutually orthogonal relationship.

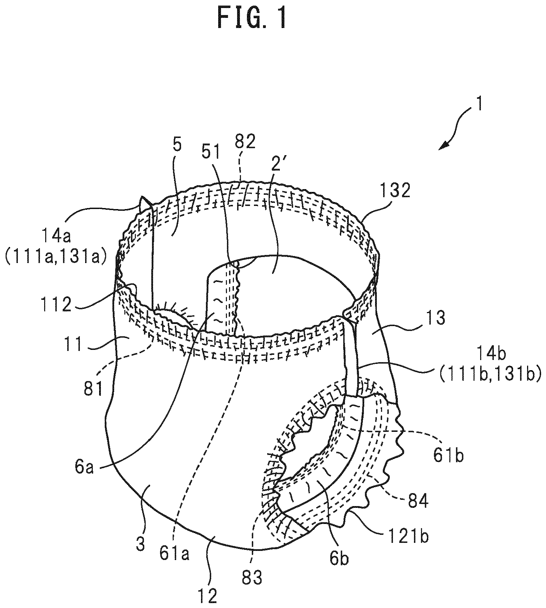

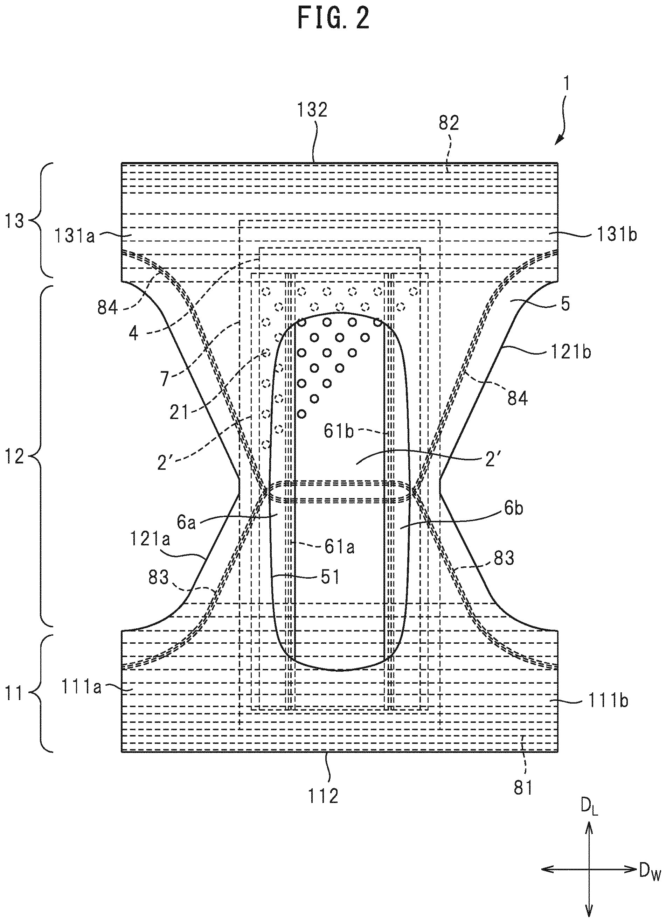

Fig. 1 is a perspective view of a disposable diaper 1 using a nonwoven fabric 2, as an embodiment of the present invention, as the top sheet 2', and Fig. 2 is a plan view of the disposable diaper 1 of Fig. 1 in the deployed state. As shown in Fig. 1 and Fig. 2, the disposable diaper 1 has a pants-type shape comprising a front section 11 which is contacted with the abdominal region of the wearer, a middle section 12 which is contacted with the crotch region of the wearer, and a back section 13 which is contacted with the gluteal region and/or back region of the wearer. In the disposable diaper 1, both side sections 111a, 111b of the front section 11 and both side sections 131a, 131b of the back section 13 are joined together at the joining sections 14a, 14b, whereby a waist opening is formed by the edge 112 of the front section 11 and the edge 132 of the back section 13, and leg openings are also formed by both side sections 121a, 121b of the middle section 12.

As shown in Fig. 1 and Fig. 2, the disposable diaper 1 comprises a liquid-permeable top sheet 2' comprising a nonwoven fabric 2 as an embodiment of the present invention, a liquid-impermeable back sheet 3, an absorbent body 4 provided between these sheets, a liquid-impermeable cover sheet 5, liquid-impermeable leak-resistant cuffs 6a, 6b, a liquid-impermeable leak-resistant sheet 7, and elastic members 81, 82, 83, 84. The cover sheet 5 provided on the skin side surface of the top sheet 2' has an opening 51 formed at approximately the center, a portion of the top sheet 2' (the portion of the region where the absorbent body 4 is situated) being exposed from the opening 51 of the cover sheet 5 and, together with the cover sheet 5, constituting the skin side surface of the disposable diaper 1. Also, at the edges of the leak-resistant cuffs 6a, 6b provided on both sides of the opening 51 of the cover sheet 5, there are provided elastic sections 61a, 61b extending in the lengthwise direction D_{L} of the disposable diaper 1.

In the disposable diaper 1, the top sheet 2' is constructed of a nonwoven fabric 2 comprising a base 22 having a first surface 2a that faces the skin surface of the wearer and a second surface 2b on the opposite side from the first surface 2a, and extending out in an approximately flat manner, and a plurality of protrusions 21 protruding in the thickness direction D_{T} from the first surface 2a of the base 22, and it is disposed on the skin side of the absorbent body 4 with the machine direction (MD direction) of the nonwoven fabric 2 being parallel to the lengthwise direction D_{L} of the disposable diaper 1. According to the present invention, the direction in which the top sheet is disposed is not limited to this aspect, and for example, the top sheet may be disposed so that the MD direction of the nonwoven fabric composing the top sheet is parallel to the widthwise direction D_{W} of the absorbent article.

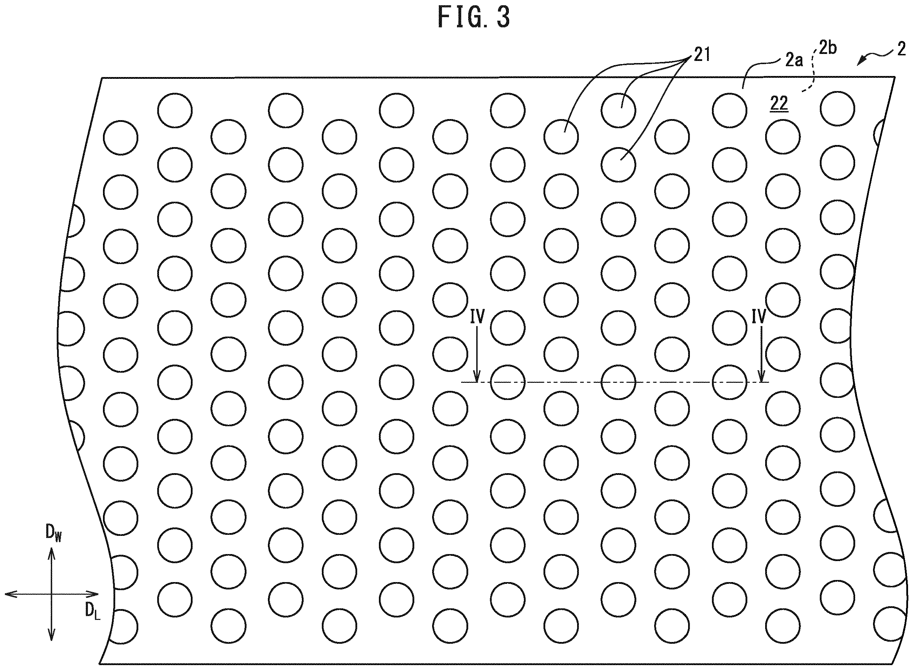

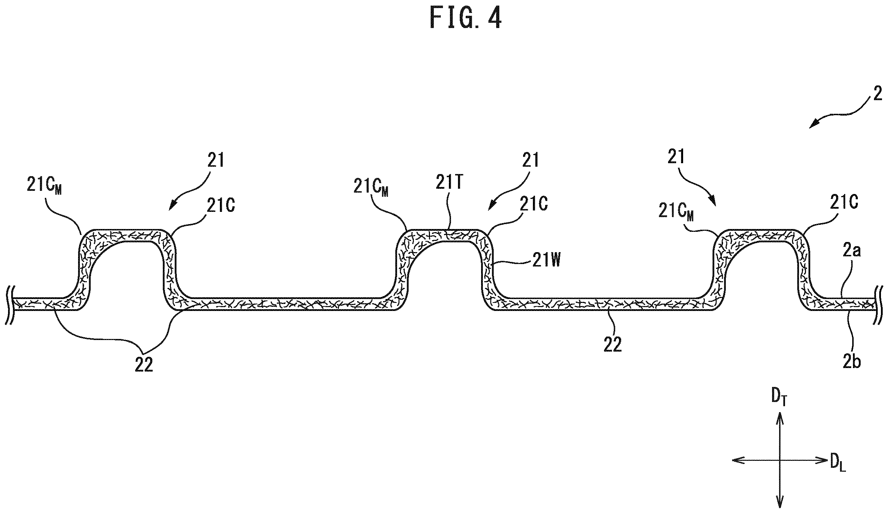

The nonwoven fabric 2 according to an embodiment of the present invention will now be explained in further detail with reference to the accompanying drawings. Fig. 3 is a partial plan view of a nonwoven fabric 2 according to an embodiment of the present invention, and Fig. 4 is a partial end view of the end face along line IV-IV of Fig. 3.

As shown in Fig. 2 to Fig. 4, the nonwoven fabric 2 of this embodiment has a long rectangular planar shape in the MD direction, and when used in a disposable diaper 1, it is disposed on the skin side of the absorbent body 4 so that the lengthwise direction of the nonwoven fabric 2 and the widthwise direction that is orthogonal to the lengthwise direction in the planar view are parallel to the lengthwise direction D_{L} and widthwise direction D_{W} of the disposable diaper 1, respectively. For simplicity in the following explanation, the lengthwise direction and widthwise direction of the nonwoven fabric 2 are referred to similarly as for the disposable diaper 1.

As shown in Fig. 3, the nonwoven fabric 2 of this embodiment has a plurality of protrusions 21 disposed so that the protrusions that are adjacent in the lengthwise direction D_{L} in the planar view do not overlap in the lengthwise direction D_{L} (i.e., it is in a zigzag arrangement). In the nonwoven fabric of the present invention, however, there is no limitation to such an arrangement, and depending on the desired cushioning properties, the plurality of protrusions may be arranged in such a manner that, for example, the protrusions that are adjacent in the lengthwise direction D_{L} in the planar view overlap in the lengthwise direction D_{L}. Also, as shown in Fig. 3, the nonwoven fabric 2 of this embodiment may have a plurality of protrusions provided at fixed intervals in the lengthwise direction D_{L} and widthwise direction D_{W} in the planar view; however, the interval between the plurality of protrusions in the nonwoven fabric of the present invention is not particularly restricted, and the protrusions may be provided at non-fixed intervals in both the lengthwise direction D_{L} and widthwise direction D_{W}. Incidentally, the interval between protrusions is the distance between prescribed points on the top surfaces corresponding to the top surface shapes of the protrusions (for example, the center points of the top surface shapes).

For this embodiment, as shown in Fig. 3 and Fig. 4, the protrusions 21 of the nonwoven fabric 2 have approximately cylindrical three-dimensional shapes comprising peripheral surface sections 21W that stand up in the thickness direction D_{T} from the first surface 2a of the base 22, and top surface sections 21T that form the crowns of the protrusions 21 and have top surfaces with circular planar shapes; however, the nonwoven fabric of the present invention is not limited to such shapes, and for example, the protrusions may be formed with any desired three-dimensional shapes that have top surfaces, including columnar, such as elliptic cylindrical or polygonal columnar, and truncated conical such as truncated conic or truncated pyramidal. Of these, from the viewpoint of feel on the skin and permeability to liquids, the protrusions preferably have cylindrical, elliptic cylindrical or truncated conic three-dimensional shapes.

According to the present invention, the height of the protrusions (i.e., the distance between the first surface of the base and an imaginary plane parallel to the first surface that includes the points at the top surface sections that are most distant from the first surface of the base), is not particularly restricted so long as it does not interfere with the effect of the present invention, but from the viewpoint of cushioning properties and feel on the skin, they are in the range of, for example, 0.1 mm to 6.0 mm, preferably 0.2 mm to 5.0 mm and even more preferably 0.2 mm to 4.0 mm.

Moreover, as shown in Fig. 3 and Fig. 4, the nonwoven fabric 2 of this embodiment comprises a base 22 that has a first surface 2a and a second surface 2b that are on mutually opposite sides and that extends out approximately flat, and a plurality of protrusions 21 that protrude in the thickness direction D_{T} from the first surface 2a of the base 22. Also, each of the plurality of protrusions 21 comprises a peripheral surface section 21W that stands up approximately in the thickness direction D_{T} from the first surface 2a of the base 22, and a top surface section 21T having a top surface forming the crown of the protrusion 21, the fiber density at the peripheral surface section 21W being higher than the fiber density at the top surface section 21T, and at least part of the top surface perimeter portion 21C of the top surface section 21T being formed as the thickest section 21C_{M} that has the greatest thickness in the nonwoven fabric 2.

The nonwoven fabric 2 constructed in this manner is formed with the fiber density of the peripheral surface sections 21W that stand up in the thickness direction D_{T} of the protrusions 21 being higher than the fiber density of the top surface sections 21T, and therefore even when pressure by body pressure or the like is applied in the thickness direction D_{T} of the nonwoven fabric 2, since the peripheral surface sections 21W function as columns supporting the top surface sections 21T, the protrusions 21 are less likely to collapse, and even if the top surface sections 21T of the protrusions 21 collapse, since at least parts of the top surface perimeter portions 21C of the top surface sections 21T are constructed as the thickest sections 21C_{M} having the greatest thickness in the nonwoven fabric 2, it is possible for collapse (deformation) of the top surface sections 21T to be restored by the resiliency of the thickest sections 21C_{M}. This allows the nonwoven fabric 2 of this embodiment to adequately exhibit the satisfactory cushioning properties exhibited by the protrusions 21, for long periods. In addition, since the thickest sections 21C_{M} have satisfactory resiliency and flexibility, even if the protrusions 21 have peripheral surface sections 21W with high fiber density, the hardness of the protrusions 21 is less likely to be felt and a satisfactory feel on the skin can be provided.

Incidentally, throughout the present description, the thickness of the nonwoven fabric is the degree of thickness of the nonwoven fabric at any point on the first surface, represented by the shortest distance between the first surface and the second surface on the opposite side. Consequently, the height of the protrusions (i.e., the shortest distance between the first surface of the base and an imaginary plane including the top surfaces of the top surface sections) is clearly distinguished from the thickness of the nonwoven fabric.

Furthermore, throughout the present description, the term "fiber density" refers to the number of cut cross-sections of the fiber per unit area when a cut surface of the nonwoven fabric is observed under magnification, and it can be measured according to the [Method of measuring fiber density] below.

The protrusions in the nonwoven fabric of the present invention will now be described in detail.

### [Top surface sections]

As shown in Fig. 3 and Fig. 4, in the nonwoven fabric 2 of this embodiment, the top surface sections 21T of the protrusions 21 have circular planar shapes at the top surfaces. If the planar shapes of the top surfaces are circular, the top surface sections 21T will be supported uniformly along the top surface perimeter portions 21C by the peripheral surface sections 21W, and therefore the sections with high local collapsability are less likely to form in the protrusions 21 (particularly the top surface sections 21T) and the protrusions 21 will be less likely to collapse, compared to other planar shapes. For this embodiment, the planar shapes of the top surfaces are circular with diameters of approximately 10 mm; however, according to the present invention the sizes of the top surfaces are not particularly restricted, and any size may be used in consideration of the desired feel on the skin, as well as strength and liquid permeability. Also, throughout the present description, the top surface perimeter portions are the perimeter sections along the outlines of the planar shapes of the top surfaces.

As shown in Fig. 4, the top surface sections 21T of the protrusions 21 for this embodiment have an essentially flat structure; however, according to the present invention there is no limitation to such a structure, and the top surface sections may have a structure in which the center sections of the top surfaces are bulging in a convex manner in the thickness direction, or they may have a structure in which the top surfaces are depressed in the thickness direction. When the top surface sections have the former structure, the cushioning property of the protrusions is greater and the feel on the skin is softer. If the top surface sections have the latter type of structure, then when pressure has been applied in the thickness direction from the first surface sides of the protrusions by body pressure or the like, since the pressure is mainly applied on the sections of the top surfaces supported by the peripheral surface sections (i.e., the top surface perimeter portions), it is less likely for the protrusions as a whole to collapse by the top surfaces deforming first.

In the nonwoven fabric of the present invention, the thicknesses of the top surface sections of the protrusions are not particularly restricted so long as they are constructed so as to have the thickest sections at least at parts of the top surface perimeter portions, and any thickness may be used, as for the peripheral surface sections of the protrusions, or the base. From the viewpoint of the cushioning property, flexibility, feel on the skin, anti-rewetting property and liquid permeability of the nonwoven fabric, they are, for example, in the range of 0.1 mm to 10.0 mm and preferably in the range of 0.2 mm to 8.0 mm. In particular, the thicknesses at the thickest sections are in the range of 0.1 mm to 10.0 mm, for example, and preferably in the range of 0.3 mm to 8.0 mm.

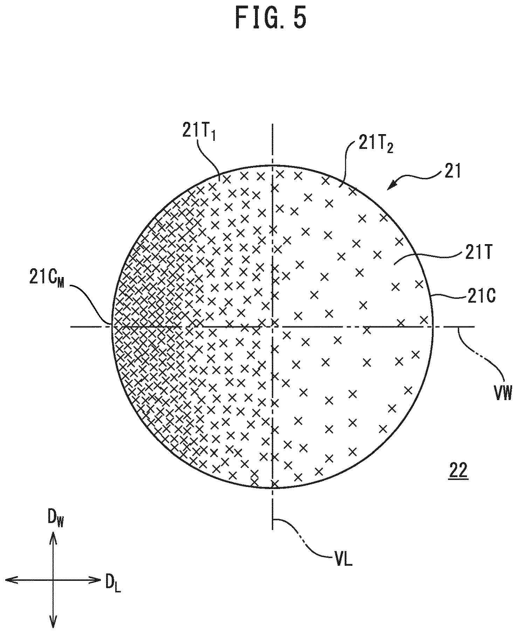

Fig. 5 is a schematic view for illustration of thickness distribution in the top surface section 21T of a protrusion 21 of a nonwoven fabric 2 according to an embodiment of the present invention. Fig. 5 shows the top surface of one protrusion 21. The "×" symbols in the drawing schematically indicate the relatively thick sections, the distribution of the thickness of the top surface section 21T being represented by the degree of the density (number) of the "×" symbols. In Fig. 5, a larger density (number) of "×" symbols corresponds to a greater thickness.

As shown in Fig. 5, for this embodiment, the top surface section 21T of the protrusion 21 is constructed so that the thickness of the top surface section 21T is maldistributed in one side in the lengthwise direction D_{L} of the nonwoven fabric 2 in the planar view. That is, of the two crossing sections where the center axis line VW on the top surface, extending in the lengthwise direction D_{L}, crosses with the top surface perimeter portion 21C in the planar view at the top surface section 21T, the crossing section located on one side in the lengthwise direction D_{L} is constructed as the thickest section 21C_{M} having the greatest thickness, while the crossing section located on the other side in the lengthwise direction D_{L} is constructed as the section with relatively small thickness. Furthermore, according to this embodiment as shown in Fig. 5, the construction is such that the thickness gradually decreases from the thickest section 21C_{M} toward the section with relatively small thickness, in a manner parallel to the center axis line VL of the top surface extending in the widthwise direction.

Also for this embodiment, the construction is such that the thickness of the top surface section 21T is maldistributed in one side in the lengthwise direction D_{L} of the nonwoven fabric 2 in the planar view; however, according to the present invention there is no limitation to this construction, and for example, the construction may be such that the thickness of the top surface section 21T is maldistributed in any one direction in the widthwise direction D_{W} of the nonwoven fabric in the planar view. Moreover, for this embodiment, all of the protrusions 21 have a similar distribution for the top surface section 21T; however, according to the present invention there is no limitation to this aspect, and for example, all of the protrusions may have different thickness distributions, or only some of the protrusions of the plurality of protrusions may have different thickness distributions. By thus adjusting the thickness distributions of the protrusions, it is possible to adjust the flow of fluid permeating through the nonwoven fabric.

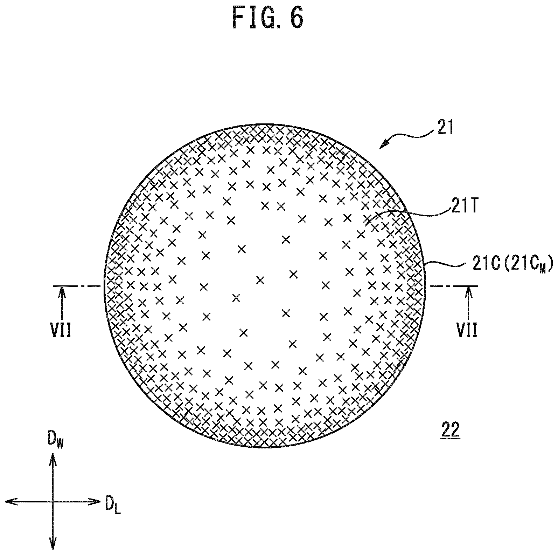

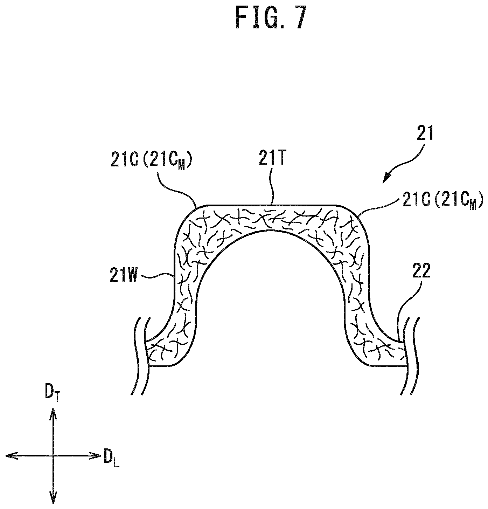

According to yet another embodiment of the present invention, the construction may be such that the thicknesses of the top surface sections 21T are greater along the top surface perimeter portions 21C in the planar view. Fig. 6 is a schematic view for illustration of thickness distribution in the top surface section 21T of a protrusion 21 of a nonwoven fabric according to another embodiment of the present invention, and Fig. 7 is an end view of the end face along line VII-VII of Fig. 6. As shown in Fig. 6 and Fig. 7, in this different embodiment of the present invention, the entire region of the top surface perimeter portion 21C of the protrusion 21 in the planar view (i.e., the entire periphery of the top surface) is constructed as the thickest section 21C_{M} that has the greatest thickness, with the thickness gradually decreasing from the entire region of the top surface perimeter portion 21C toward the center of the circular top surface. When the protrusions of the nonwoven fabric are constructed in this manner, the thickest sections that have satisfactory resiliency and flexibility are formed along the entire periphery of the top surfaces of the protrusions, and therefore the hardness of the protrusions is even less likely to be felt and a satisfactory feel on the skin can be obtained, while the cushioning property can also be improved.

In the nonwoven fabric of the present invention, the fiber density at the top surface sections of the protrusions is not particularly restricted so long it satisfies the condition of having lower fiber density than the peripheral surface sections without interfering with the satisfactory cushioning property and feel on the skin, and they may have any desired fiber density. Such a fiber density is in the range of 20 num/mm² to 200 num/mm², for example, and preferably in the range of 30 num/mm² to 150 num/mm², where one fiber cross-section is assumed to be a single fiber.

In the nonwoven fabric of the present invention, the thickest sections of the protrusions preferably have the lowest fiber density in the nonwoven fabric. If the thickest sections have the lowest fiber density in the nonwoven fabric, the resiliency and flexibility of the thickest sections will be relatively high, and therefore even when the top surface sections of the protrusions collapse, the collapse (deformation) of the top surface sections can be reliably restored, the hardness of the protrusions is even less likely to be felt, and a more satisfactory feel on the skin can be provided.

### [Peripheral surface sections]

According to this embodiment, the peripheral surface sections 21W of the protrusions 21 are the sections corresponding to the sides of the essentially cylindrical protrusions 21, and they stand up in the thickness direction D_{T} from the first surface 2a of the base 22 that extends flat and have an essentially tubular structure extending in the thickness direction D_{T}, being connected to the top surface sections 21T at the edges on the side opposite the base 22. Furthermore, although the angle formed between the first surface 2a of the base 22 and the outer surface of the peripheral surface section (hereunder referred to as "standing angle") is approximately 90° for this embodiment as shown in Fig. 4, according to the present invention there is no limitation to this angle, and the standing angle may be an angle exceeding 90° or an angle less than 90°. If the standing angle is an angle exceeding 90°, the peripheral surface sections will be able to more firmly support the top surface sections, so that the protrusions can be even less likely to collapse. If the standing angle is less than 90°, the protrusions will tend to deform in the in-plane direction of the nonwoven fabric, and therefore even when pressure is applied in the in-plane direction of the nonwoven fabric (for example, when the skin surface moves along the surface of the nonwoven fabric), a satisfactory feel on the skin can be provided.

According to the present invention, the thicknesses of the peripheral surface sections of the protrusions are not particularly restricted so long as the function as columns supporting the top surface sections is not inhibited, and any thickness may be used, as for the top surface sections of the protrusions, or the base. From the viewpoint of the resistance to collapse of the protrusions and the cushioning property, flexibility, feel on the skin, anti-rewetting property and liquid permeability, they are, for example, in the range of 0.1 mm to 10.0 mm and preferably in the range of 0.3 mm to 7.0 mm. Moreover, the peripheral surface sections may be constructed so as to have uniform thickness across the entirety, or they may be constructed so that the thickness gradually increases from the base side toward the top surface section side. In particular, when the peripheral surface sections are constructed so that the thickness gradually increases from the base side toward the top surface section side, the thickness at the parts of the peripheral surface sections connected to the top surface perimeter portions of the top surface sections (especially the thickest sections) is greater, increasing the resiliency and flexibility at those parts, and therefore collapse (deformation) of the top surface sections can be more reliably restored.

In the nonwoven fabric of the present invention, the fiber density at the peripheral surface sections of the protrusions is not particularly restricted so long it satisfies the condition of having higher fiber density than the top surface sections without interfering with the satisfactory cushioning property and feel on the skin, and they may have any desired fiber density. Such a fiber density is in the range of 30 num/mm² to 150 num/mm², for example, and preferably in the range of 50 num/mm² to 120 num/mm². Furthermore, the fiber density of the peripheral surface sections is preferably higher than the fiber density of the base. If the fiber density of the peripheral surface sections is higher than the fiber density of the base, pressure in the thickness direction applied from the first surface sides of the protrusions can be buffered by bending of the base to the second surface side, and therefore the protrusions will be even less likely to collapse and excellent flexibility can be imparted to the nonwoven fabric.

The construction other than the protrusions of the nonwoven fabric of the present invention will now be described.

### [Base]

In the nonwoven fabric of the present invention, the base has a first surface with the aforementioned protrusions and a second surface on the opposite side from the first surface, and it is the section extending essentially flat. The thickness of the base is not particularly restricted so long as it does not interfere with the satisfactory cushioning property, flexibility and liquid permeability, and any desired thickness may be employed. From the viewpoint of the cushioning property, flexibility, feel on the skin, anti-rewetting property and liquid permeability of the nonwoven fabric, it is in the range of 0.10 mm to 10.0 mm, for example, or in the range of preferably 0.15 mm to 7.0 mm and even more preferably 0.20 mm to 5.0 mm.

In the nonwoven fabric of the present invention, the fiber density of the base is not particularly restricted so long as it does not interfere with the satisfactory cushioning property, flexibility and liquid permeability, and it may have any desired fiber density. Such a fiber density is in the range of 10 num/mm² to 150 num/mm², for example, and preferably in the range of 20 num/mm² to 100 num/mm².

Furthermore, the fiber density of the base is preferably lower than the fiber density of the peripheral surface sections of the protrusions. If the fiber density of the base is lower than the fiber density of the peripheral surface sections of the protrusions, pressure in the thickness direction applied from the first surface sides of the protrusions can be buffered by bending of the base to the second surface side, and therefore the protrusions will be even less likely to collapse and excellent flexibility can be imparted to the nonwoven fabric. Furthermore, according to the present invention, the fiber density of the base is preferably lower than the fiber density of the top surface sections of the protrusions. If the fiber density of the base is lower than the fiber density of the top surface sections of the protrusions, pressure in the thickness direction applied from the first surface sides of the protrusions can be buffered by bending of the base to the second surface side before the top surface sections of the protrusions deform (collapse), and therefore the protrusions will be even much less likely to collapse and even more excellent flexibility can be imparted to the nonwoven fabric.

### [Nonwoven fabric entirety]

According to the present invention, the basis weight of the nonwoven fabric is not particularly restricted but is preferably 10 g/m² to 100 g/m² and more preferably 15 g/m² to 50 g/m². If the basis weight is 10 g/m² or greater, sufficient surface strength as a nonwoven fabric will be obtained, and therefore when the nonwoven fabric has been applied to the top sheet of an absorbent article, for example, the top sheet will be less likely to tear during use of the absorbent article. Also, if the basis weight is 100 g/m² or lower, stiffness will not be produced, and when the nonwoven fabric has been applied in a top sheet of an absorbent article, for example, the wearer will be less likely to have unpleasantness or discomfort occur during use of the absorbent article.

The fibers to be used in the nonwoven fabric of the present invention are not particularly restricted so long as they do not inhibit the satisfactory cushioning property and feel on the skin, and examples include natural fibers such as wool and cotton; regenerated fibers such as rayon and acetate; synthetic fibers composed using thermoplastic resins alone, including polyolefins such as polyethylene (PE) and polypropylene (PP); ethylene-vinyl acetate copolymer (EVA); ethylene-ethyl acrylate copolymer; ethylene-acrylic acid copolymer; ionomers; polyesters such as polyethylene terephthalate (PET), polybutylene terephthalate (PBT) and polylactic acid, and polyamides such as nylon, or composite fibers such as core-sheath, side-by-side or sea-island fibers composed using multiple different types of these thermoplastic resins. Core-sheath composite fibers preferably have a core-sheath structure wherein the core is polyethylene terephthalate (PET) and the sheath is polyethylene such as high-density polyethylene (HDPE) or low melting point polypropylene, and representative examples of such composite fibers include composite fibers with core/sheath = PET/HDPE, PET/PE, PP/PE and PP/low melting point PP. The form of such fibers is not particularly restricted, and fibers in the form of hollow fibers; irregular cross-sectional fibers that are flat, Y-shaped or the like, latent crimping or developed crimping type solid crimped fibers, or splittable fibers that split under a physical load such as a water stream, heat or embossing, may be suitably used. Such fibers may also be subjected to hydrophilicizing treatment. Each of the fibers mentioned above may be used alone, or two or more may be used in desired combinations.

A nonwoven fabric having a plurality of protrusions according to the present invention can be produced by using a nonwoven fabric produced by any desired production method, such as an air-through nonwoven fabric, spunlace nonwoven fabric, spunbond nonwoven fabric, thermal bond nonwoven fabric or meltblown nonwoven fabric, as the base material, and forming the plurality of protrusions on the surface of the base material; however, for more precise production of a nonwoven fabric having the plurality of protrusions, it is preferably produced by the production method described below.

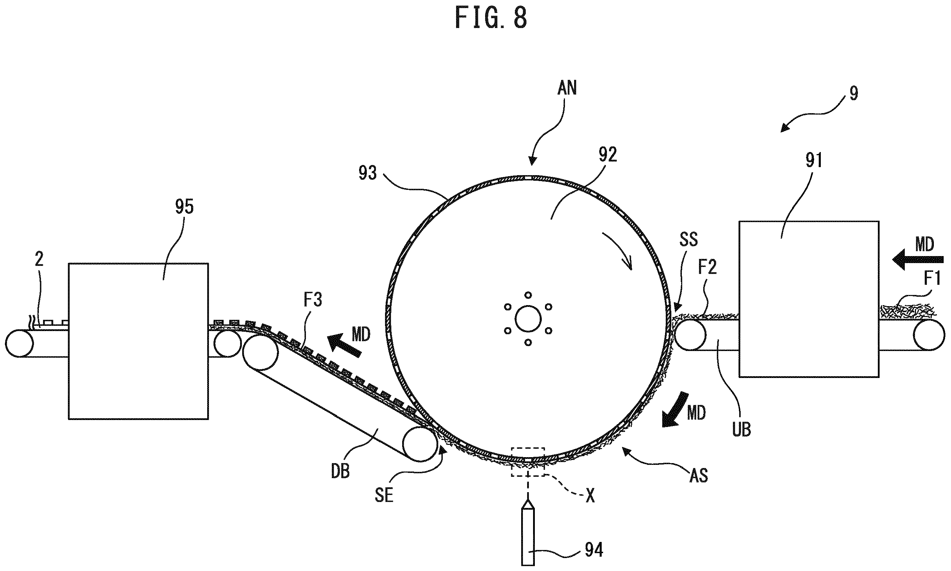

An example of a production method for a nonwoven fabric according to the present invention will now be explained with reference to Fig. 8.

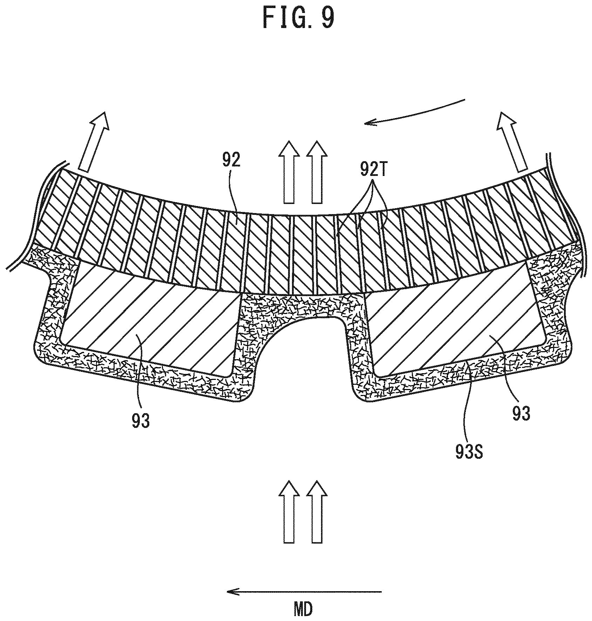

Fig. 8 is a schematic diagram showing an example of a production apparatus 9 for production of a nonwoven fabric with a plurality of protrusions according to the present invention, and Fig. 9 is a magnified schematic view of section X of Fig. 8. As shown in Fig. 8 and Fig. 9, the production apparatus 9 comprises a carding machine 91 that opens the fibers F1 as the starting material, while adjusting the basis weight, a shaping apparatus that includes a suction drum 92 and an air jet nozzle 94, that shapes the plurality of protrusions in the fiber matrix F2 that has left the carding machine 91, and a heat setting machine 95 that heat sets the fiber matrix F3 having the plurality of protrusions shaped by the shaping apparatus, to fix the structure of the protrusions. In Fig. 8, the fibers F1, fiber matrix F2, fiber matrix F3 and the nonwoven fabric 2 with the plurality of protrusions are conveyed continuously in the MD direction indicated by the arrows in Fig. 8. The MD direction matches the lengthwise direction D_{L} of the nonwoven fabric 2.

In the method for producing a nonwoven fabric using this type of production apparatus 9, first the fibers F1 such as thermoplastic fibers or natural fibers, as the starting material, are supplied to the carding machine 91. At the carding machine 91, the supplied fibers F1 are opened and the basis weight of the fibers F1 is adjusted to the prescribed value.

The fiber matrix F2 that has left the carding machine 91 is then conveyed toward the surface of the suction drum 92. The suction drum 92 preferably has its interior formed in a hollow or porous manner, and the interior may be brought to a negative pressure by any desired suction means such as a vacuum pump. Also, as shown in Fig. 9, a plurality of suction holes 92T are formed on the outer peripheral surface of the suction drum 92, and external air is suctioned near the outer peripheral surface through the suction holes 92T. Furthermore, the suction holes 92T are formed to sizes that prevent the fiber matrix F2 from being sucked inside the suction drum, while ensuring the desired suction force.

In addition, the outer peripheral surface of the suction drum 92 is covered by a pattern plate 93, and the fiber matrix F2 that has left the carding machine 91 is fed onto the pattern plate 93 that is rotating with the suction drum 92. The pattern plate 93 is composed of punching metal in which through-holes are formed in a prescribed pattern, with shapes complementary to the protrusions 21 of the nonwoven fabric 2 that will have a plurality of protrusions 21. Furthermore, as shown in Fig. 9, the fiber matrix F2 supplied onto the pattern plate 93 is suctioned by negative pressure from the suction holes 92T formed in the outer peripheral surface of the suction drum 92, and is attracted onto the surface 93S of the pattern plate 93 and in the through-holes.

Also, as shown in Fig. 8, the suction drum 92 is constructed so that it sucks the fiber matrix at least in a region AS between a point SS on the outer peripheral surface on which the fiber matrix F2 is supplied from a belt conveyor UB situated at the upstream end, and a point SE on the outer peripheral surface where the fiber matrix F3 is supplied onto a belt conveyor DB situated at the downstream end. Incidentally, the construction is preferably such that no suction takes place in the region AN outside of the region AS, from the viewpoint of the suction efficiency of the suction drum 92 and preventing inclusion of contaminants.

As shown in Fig. 8 and Fig. 9, the fiber matrix F2 that has been adsorbed onto the pattern plate 93 on the outer peripheral surface of the suction drum 92 is blasted with hot air by the air jet nozzle 94. The air jet nozzle 94 is composed of one spray hole, or a plurality aligned in the widthwise direction of the fiber matrix F2, allowing hot air at a prescribed temperature to be sprayed at a prescribed air speed. Therefore, in the shaping apparatus, suction with the suction drum 92 and blasting with hot air by the air jet nozzle 94 allows the protrusions 21 having the structure specified above in the nonwoven fabric 2 (i.e., a structure in which the fiber density of the peripheral surface sections of the protrusions is higher than the fiber density of the top surface sections, and at least parts of the top surface perimeter portions of the top surface sections are constructed as the thickest sections having the greatest thickness in the nonwoven fabric), to be shaped into the fiber matrix F2.

The temperature and wind speed of the hot air blasted from the air jet nozzle 94 are each set to conditions so that protrusions having the structure specified above are formed, in consideration of the type of fibers composing the fiber matrix and the basis weight of the fiber matrix. The temperature conditions for the hot air are preferably set to be 20°C to 70°C higher than the melting point of the fibers composing the fiber matrix. For example, when the fibers composing the fiber matrix are PET/HDPE core-sheath composite fibers, the temperature of the hot air is preferably in the range of 80°C to 400°C. The wind speed conditions for the hot air are in the range of 10.0 m/sec to 200.0 m/sec, for example, and preferably in the range of 20.0 m/sec to 150.0 m/sec, in consideration of the basis weight and thickness of the nonwoven fabric.

Also, as shown in Fig. 8, the fiber matrix F3 having the plurality of protrusions 21 shaped by the suction drum 92 and air jet nozzle 94 is then conveyed to the heat setting machine 95 by the belt conveyor DB, and subjected to heat setting in the heat setting machine 95. The heat setting fixes the structure of the plurality of protrusions 21 shaped in the fiber matrix F3, and imparts flexibility to the nonwoven fabric 2. The temperature for the heat setting is not particularly restricted, and may be in the range of 100°C to 150°C, for example.

When heat setting of the fiber matrix F3 has been completed in this manner, a nonwoven fabric according to the present invention is obtained. The obtained nonwoven fabric may be cut to a desired size, according to the form in which it is to be used.

The nonwoven fabric of the present invention may be applied to any desired product that requires satisfactory cushioning properties and feel on the skin, and for example, it may be suitably used in an absorbent article such as a disposable diaper of the embodiment described above. Particularly when used as the top sheet of an absorbent article, the nonwoven fabric of the present invention can adequately exhibit satisfactory cushioning properties for long periods and can also provide a satisfactory feel on the skin, and therefore the wearer of the absorbent article can be provided with an excellent feel during wear for long periods.

Incidentally, an absorbent article to which the nonwoven fabric of the present invention is applied may be any of various types of absorbent articles, such as a pants-type disposable diaper according to the embodiment described above, or a sanitary napkin, tape-type disposable diaper or incontinence pad, for example. In addition, the absorbent article of the present invention is not restricted to the embodiments described above and the examples described below, and can incorporate appropriate combinations and modifications within a range that is not outside of the object and gist of the present invention.

### Examples

The present invention will now be explained in greater detail using examples and comparative examples, with the understanding that the present invention is not limited only to the examples.

### Example 1

After opening PET/HDPE core-sheath composite fibers with a fineness of 1.3 dtex using an opener, the fibers were formed using a carding machine. The formed carded web was conveyed by a mesh conveyor, and supplied onto a pattern plate mounted on the outer peripheral surface of the suction drum. While rotating the suction drum, the carded web was suctioned into the through-holes of the pattern plate by negative pressure from suction holes formed in the outer peripheral surface of the suction drum, and the carded web was blasted with hot air at 180°C at a wind speed of 50.0 m/sec, from an air jet nozzle situated on the lower side of the suction drum, thereby causing the carded web to run along the pattern plate to shape a plurality of protrusions on the carded web.

Also, the web on which the plurality of protrusions had been shaped was conveyed to a heat setting machine and subjected to heat setting under hot air conditions with a temperature of 135°C and a wind speed of 0.9 m/sec, after which the heat set web was wound up to obtain a nonwoven fabric for Example 1. The basis weight of the obtained nonwoven fabric was 25 g/m².

### Comparative Example 1

A nonwoven fabric for Comparative Example 1 was obtained in the same manner as Example 1, except that the temperature and wind speed of the hot air blasted from the air jet nozzle were 143°C and 29.2 m/sec, respectively. The basis weight of the obtained nonwoven fabric was 25 g/m².

The cross-sectional structures of the protrusions (the cross-sectional structures along the center axis line VW in Fig. 5) of each of the nonwoven fabrics obtained in Example 1 and Comparative Example 1 were photographed with a scanning electron microscope, and the fiber densities of the top surface sections and peripheral surface sections of the protrusions and of the bases were measured by the measuring method described below. Also, for the nonwoven fabrics of Example 1 and Comparative Example 1, the specific volume under compression per protrusion was measured by the measuring method described below in order to evaluate the compressive strength of the protrusions. The feel on the skin of the protrusion surfaces of the nonwoven fabrics of Example 1 and Comparative Example 1 were also confirmed by tactile perception.

Measurement of the fiber density was carried out in the same manner as the method described in Japanese Unexamined Patent Publication No. 2012-144835 (see paragraph [0041]). Specifically, the fiber density was measured according to the following [Method of measuring fiber density].

### [Method of measuring fiber density]

A cut surface of the nonwoven fabric is observed in an enlarged view using a scanning electron microscope (for example, a VE-7800 Real Surface View microscope by Keyence Corp.), with the center in the thickness direction of the nonwoven fabric as the center of observation (i.e., observation at a magnification allowing about 20 to 70 fiber cross-sections to be observed, which is normally a magnification of about 20x to 100x), and the number of cut cross-sections of fibers per fixed area (about 2 mm²) is counted. The counted number of cut cross-sections is converted to the number of cut cross-sections of fibers per 1 mm², and recorded as the fiber density (num/mm²). This fiber density measurement is carried out at 3 locations, and the mean value is recorded as the fiber density for the sample.

### [Method of measuring specific volume under compression per protrusion]

Ten sample sheets are prepared by cutting the nonwoven fabric with a plurality of protrusions into 100 mm × 100 mm quadrilateral shapes. The 10 prepared sample sheets are stacked and set on the measuring stage of an "Autograph AGS-1kNG" by Shimadzu Corp. One cycle of the compression test is carried out, under test conditions of chuck distance: 35 mm, compression speed: 25 mm/min, maximum load: 26 N. The sample sheet is compressed, the thickness (cm) of the sample sheet when the chuck distance has shortened is measured, and this thickness is divided by the sample sheet basis weight (g/cm²) to obtain the specific volume under compression (cm³/g) for the sample sheet. Also, the obtained specific volume under compression is divided by the number of protrusions in the sample sheet, and the resulting value is recorded as the specific volume under compression (cm³/g/protrusion) for each protrusion of the nonwoven fabric with the plurality of protrusions.

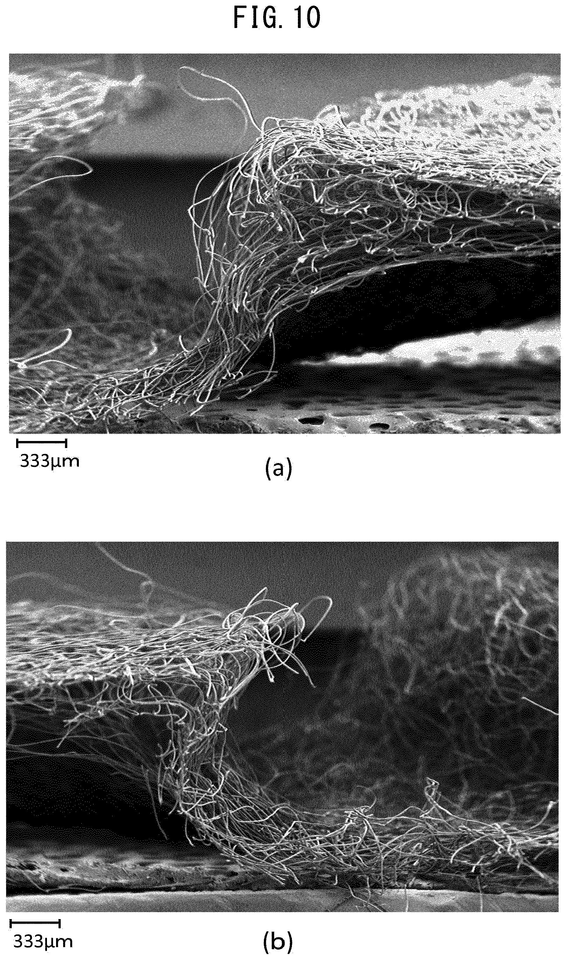

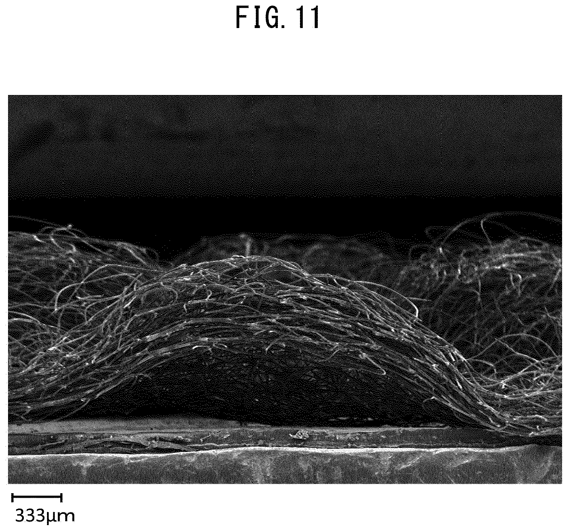

An electron micrograph of a cross-section of a protrusion of the nonwoven fabric of Example 1 is shown in Fig. 10, and an electron micrograph of a cross-section of a protrusion of the nonwoven fabric of Comparative Example 1 is shown in Fig. 11. Fig. 10(a) is a photomicrograph of a cross-section along the center axis line in the MD direction at a section of the protrusion at the downstream end in the MD direction (a line corresponding to the center axis line VW in Fig. 5), and Fig. 10(b) is a photomicrograph of a cross-section along the center axis line in the MD direction at a section of the protrusion at the upstream end in the MD direction.

Table 1 shows the measurement results for the fiber density of the protrusions of the nonwoven fabrics of Example 1 and Comparative Example 1, and the specific volume under compression per protrusion.

**[Table 1]**

| | | Example 1 | Comparative Example 1 |
|---|---|---|---|
| Fiber density | Top surface sections (num/mm²) | 59 | 38 |
| | Peripheral surface sections (num/mm²) | 95 | 23 |
| | Base (num/mm²) | 46 | 45 |
| Specific volume under compression per protrusion (cm³/g/protrusion) | | 0.218 | 0.014 |

As shown in Table 1, the nonwoven fabric of Example 1 has a specific volume under compression per protrusion that is markedly larger compared to the nonwoven fabric of Comparative Example 1, indicating that the protrusions are less likely to collapse.

In addition, as shown Fig. 10(a) and (b), the nonwoven fabric of Example 1 had a specific structure in which the protrusions comprised peripheral surface sections that stood up in the thickness direction from the first surface of the base, and top surface sections having top surfaces that formed the crowns of the protrusions, with parts of the top surface perimeter portions of the top surface sections constructed as the thickest sections, and the feel on the skin of the surface with the protrusions was satisfactory. On the other hand, as shown in Fig. 11, the nonwoven fabric of Comparative Example 1 had a structure in which the protrusions were essentially hemispherical, and the feel on the skin of the surface with the protrusions was such that the protrusions were easily felt.

### Reference Sign List

- 1: Disposable diaper
- 2: Nonwoven fabric
- 2': Top sheet
- 21: Protrusion
- 21T: Top surface section
- 21W: Peripheral surface section
- 21C: Top surface perimeter portion
- 21C_{M}: Thickest section
- 22: Base
- 3: Back sheet
- 4: Absorbent body

## Claims

1. A nonwoven fabric (2) comprising a base (22) having a mutually opposite first surface (2a) and second surface (2b) and extending out flat, and a plurality of protrusions (21) protruding from the first surface (2a) of the base (22) in a thickness direction, wherein
each of the protrusions (21) comprises a peripheral surface section (21W) standing up from the first surface (2a) of the base (22) in the thickness direction and a top surface section (21T) having a top surface forming a crown of the each of the protrusions (21),
a fiber density of the peripheral surface section (21W) is higher than a fiber density of the top surface section (21T), and **characterized in that**
the top surface section (21T) is constructed so that a top surface perimeter portion (21C) located on one side in a machine direction or an orthogonal direction with respect to the machine direction in the planar view is constructed as a section with greater thickness, while the top surface perimeter portion located on the other side in the machine direction or an orthogonal direction is constructed as a section with smaller thickness, and
the section with greater thickness comprises a thickest section (21C_{M}) which has the greatest thickness in the nonwoven fabric (2).

2. The nonwoven fabric (2) according to claim 1, wherein the fiber density of the peripheral surface section (21W) is higher than fiber density of the base (22).

3. The nonwoven fabric (2) according to claim 1 or 2, wherein the top surface of the top surface section (21T) is depressed.

4. The nonwoven fabric (2) according to any one of claims 1 to 3, wherein a planar shape of the top surface of the top surface section (21T) is circular.

5. The nonwoven fabric (2) according to any one of claims 1 to 4, wherein the thickest section (21C_{M}) has the lowest fiber density in the nonwoven fabric (2).

6. An absorbent article (1) including the nonwoven fabric (2) according to any one of claims 1 to 5.

## Patentansprüche

1. Vliesstoff (2), welche eine Basis (22), die eine erste Oberfläche (2a) und eine zweite Oberfläche (2b) aufweist, die einander gegenüber liegen, wobei die Basis sich flach nach außen erstreckt, und eine Vielzahl an Vorsprüngen (21) aufweist, die von der ersten Oberfläche (2a) der Basis (22) in einer Dickenrichtung vorstehen, wobei
jeder der Vorsprünge (21) einen Umfangsflächenabschnitt (21W), der von der ersten Oberfläche (2a) der Basis (22) in der Dickenrichtung vorsteht, und einen oberen Oberflächenabschnitt (21T) mit einer oberen Oberfläche aufweist, die eine Krone aus den einzelnen Vorsprünge (21) bildet, wobei
eine Faserdichte des Umfangsoberflächenabschnitts (21W) höher als eine Faserdichte des oberen Oberflächenabschnitts (21T) ist, und der Vliesstoff **dadurch gekennzeichnet ist, dass**
der obere Oberflächenabschnitt (21T) auf eine solche Weise aufgebaut ist, dass ein oberer Oberflächenumfangsabschnitt (21C), der auf einer Seite in einer Maschinenrichtung oder einer orthogonalen Richtung in Bezug auf die Maschinenrichtung in der Draufsicht angeordnet ist, als ein Abschnitt mit einer größeren Dicke aufgebaut ist, während der Umfangsabschnitt der oberen Oberfläche, der sich in der Maschinenrichtung oder in der orthogonale Richtung auf der anderen Seite befindet, als ein Abschnitt mit einer geringeren Dicke ausgebildet ist, und dass
der Abschnitt mit einer größeren Dicke einen dicksten Abschnitt (21C_{M}) aufweist, der die größte Dicke in dem Vliesstoff (2) aufweist.

2. Vliesstoff (2) nach Anspruch 1, wobei die Faserdichte des Umfangsoberflächenabschnitts (21W) höher als die Faserdichte der Basis (22) ist.

3. Vliesstoff (2) nach Anspruch 1 oder 2, wobei die obere Oberfläche des oberen Oberflächenabschnitts (21T) vertieft ist.

4. Vliesstoff (2) nach einem der Ansprüche 1 bis 3, wobei eine planare Form der oberen Oberfläche des oberen Oberflächenabschnitts (21T) kreisförmig ist.

5. Vliesstoff (2) nach einem der Ansprüche 1 bis 4, wobei der dickste Abschnitt (21C_{M}) die niedrigste Faserdichte in dem Vliesstoff (2) aufweist.

6. Saugfähiger Artikel (1), der den Vliesstoff (2) nach einem der Ansprüche 1 bis 5 enthält.

## Revendications

1. Non-tissé (2) comprenant une base (22) ayant une première surface (2a) et une seconde surface (2b) mutuellement opposées et s'étendant à plat, et une pluralité de protubérances (21) faisant protubérance depuis la première surface (2a) de la base (22) dans une direction d'épaisseur, dans lequel
chacune des protubérances (21) comprend une section de surface périphérique (21W) s'érigeant depuis la première surface (2a) de la base (22) dans la direction d'épaisseur et une section de surface haute (21T) ayant une surface haute formant une couronne de chacune des protubérances (21),
une densité de fibres de la section de surface périphérique (21W) est plus élevée qu'une densité de fibres de la section de surface haute (21T), et **caractérisé en ce que**
la section de surface haute (21T) est construite pour qu'une portion de périmètre de surface haute (21C) située sur un côté dans une direction machine ou une direction orthogonale par rapport à la direction machine dans la vue en plan soit construite comme une section de plus grande épaisseur, alors que la portion de périmètre de surface haute située sur l'autre côté dans la direction machine ou une direction orthogonale est construite comme une section de plus petite épaisseur, et
la section de plus grande épaisseur comprend une section la plus épaisse (21C_{M}) qui a la plus grande épaisseur dans le Non-tissé (2).

2. Non-tissé (2) selon la revendication 1, dans lequel la densité de fibres de la section de surface périphérique (21W) est plus élevée que la densité de fibres de la base (22).

3. Non-tissé (2) selon la revendication 1 ou 2, dans lequel la surface haute de la section de surface haute (21T) est enfoncée.

4. Non-tissé (2) selon l'une quelconque des revendications 1 à 3, dans lequel une forme plane de la surface haute de la section de surface haute (21T) est circulaire.

5. Non-tissé (2) selon l'une quelconque des revendications 1 à 4, dans lequel la section la plus épaisse (21C_{M}) a la plus faible densité de fibres dans le Non-tissé (2).

6. Article absorbant (1) comportant le Non-tissé (2) selon l'une quelconque des revendications 1 à 5.
